# EUROPÄISCHE PATENTSCHRIFT

(11) **EP 1 799 627 B1**
(45) Veröffentlichungstag und Bekanntmachung des Hinweises auf die Patenterteilung: **05.05.2010**
(21) Anmeldenummer: 05792391.4
(22) Anmeldetag: 11.10.2005
(51) Int. Cl.: C07B 37/04, C07C 41/30, C07C 45/68, C07C 67/343, C07C 253/30, C07C 303/30, C07D 209/42, C07D 213/61, C07D 213/57, C07D 213/50, C07D 295/18, C07D 317/54, C07D 317/58, C07D 333/22, C07D 333/56

(54) **EISEN- ODER KOBALT-KATALYSIERTE KOHLENSTOFF-KOHLENSTOFF-KUPPLUNGSREAKTION VON ARYLEN, ALKENEN UND ALKINEN MIT KUPFERREAGENZIEN**
IRON OR COBALT-CATALYZED CARBON-CARBON COUPLING REACTION OF ARYLS, ALKENES AND ALKINES WITH COPPER REAGENTS
REACTION DE COUPLAGE CARBONE-CARBONE, CATALYSEE PAR DU FER OU DU COBALT, D'ARYLES, D'ALCENES ET D'ALKINES AVEC DES REACTIFS DE CUIVRE

(30) Priorität: 11.10.2004 DE 102004049508
(43) Veröffentlichungstag der Anmeldung: 27.06.2007
(73) Patentinhaber: Saltigo GmbH, 40764 Langenfeld (DE)
(72) Erfinder: KNOCHEL, Paul, 81479 München (DE); SAPOUNTZIS, Ioannis, 1120 Wien (AT); KORN, Tobias, 67227 Frankenthal (DE); LIN, Wenwei, 14197 Berlin-Wilmersdorf (DE); KOFINK, Christiane Charlotte, 81829 München (DE)
(74) Vertreter: Pettrich, Klaus-Günter
(86) Internationale Anmeldenummer: PCT/EP2005/010936
(87) Internationale Veröffentlichungsnummer: WO 2006/040131

(56) Entgegenhaltungen:
- WO-A-02/00340
- WO-A-99/51609
- US-A1- 2003 220 498
- KNOCHEL P ET AL: "SYNTHESE HOCH FUNKTIONALISIERTER ORGANOMAGNESIUM-REAGENTIEN DURCH HALOGEN-METALL-AUSTAUSCH" ANGEWANDTE CHEMIE, VCH VERLAGSGESELLSCHAFT, WEINHEIM, DE, Bd. 115, 2003, Seiten 4438-4456, XP002294440 ISSN: 0044-8249 in der Anmeldung erwähnt
- KRASOVSKIY A ET AL: "A LICL-MEDIATED BR/MG EXCHANGE REACTION FOR THE PREPARATION OF FUNCTIONALIZED ARYL- AND HETEROARYLMAGNESIUM COMPOUNDS FROM ORGANIC BROMIDES" ANGEWANDTE CHEMIE, INTERNATIONAL EDITION, WILEY VCH VERLAG, WEINHEIM, DE, Bd. 43, 26. Mai 2004 (2004-05-26), Seiten 3333-3336, XP002294441 ISSN: 1433-7851 in der Anmeldung erwähnt
- INOUE, A. ET AL.: "Selective halogen-magnesium exchange reaction via organomagnesium ate complex" J. ORG. CHEM., Bd. 66, Nr. 12, 2001, Seiten 4333-4339, XP002367355

## Beschreibung

Die vorliegende Erfindung betrifft ein Verfahren zur Knüpfung von Kohlenstoff-Kohlenstoff-Bindungen ausgehend von einer Kupferverbindung eines Aryls, Heteroalryls, Alkens oder Alkins und einer Aryl-, Heteroaryl-, Alken- oder Alkinverbindung mit einer geeigneten Abgangsgruppe. Die Kreuzkupplung dieser Verbindungen mit einer z.B. halogensubstituierten Arylverbindung erfolgt mit Hilfe eines Eisen- oder Kobalt-Katalysators unter Verwendung geeigneter Lösungsmittel.

### Hintergrund der Erfindung

Übergangsmetallkatalysierte Kreuzkupplungen sind sehr leistungsfähige Reaktionen zur Knüpfung von C-C-Bindungen, insbesondere zwischen Csp²-Zentren, an denen typische S_{N}2-Substitutionen nicht möglich sind.^{[1]} Die Aryl-Aryl-Kreuzkupplung ist eine der wichtigsten Methoden, um Kohlenstoff-Kohlenstoff-Bindungen aufzubauen. Viele der dadurch erhaltenen Aromaten und insbesondere der Heteroaromaten sind von großem Interesse sowohl für die Agro- und Pharmaindustrie als auch für die Materialwissenschaften. Hierfür werden zumeist zuverlässige Palladium(0) Katalysatoren, ^{[1,2]} vornehmlich in Gegenwart eines entsprechenden Liganden, wie beispielsweise sterisch gehinderte Phosphine, eingesetzt.^{[3]} Auch Nickel(0)-Komplexe finden nützliche Anwendungen, aber sie scheinen weniger breite Verwendungsmöglichkeiten zu haben.^{[4]} Da Palladium aber teuer und Nickel toxisch ist besteht der Bedarf an billigen und ungiftigen Katalysatoren. Eisen- und Kobaltsalze stellen solche billigen und ungiftigen Alternativen dar.

Der Arbeit von Kochi folgend,^{[5]} wurden Eisen-katalysierte Kreuzkupplungen in letzter Zeit sehr intensiv auf ihre Leistungsfähigkeit in Kreuzkupplungsreaktionen erforscht.^{[6]} Obwohl sehr effiziente Kreuzkupplungen zwischen einer Reihe von Alkylmagnesium-Reagenzien und Arylhalogeniden oder Arylsulfonaten verwirklicht wurden, blieb die katalysierte Kreuzkupplung zweier Arylreste aufgrund der beträchtlichen Homokupplungsreaktion der Arylmagnesiumspezies problematisch und es konnte bislang keine synthetische Lösung für dieses Problem gefunden werden.^{[6,7]} Aber auch die Dehalogenierung des Arylhalogenides tritt dabei auf.

Deshalb haben die Erfinder die Arylmagnesium-Spezies auf die entsprechende Organozink-Verbindung transmetalliert, welche eine verringerte Tendenz zur Bildung instabiler At-Komplexe besitzt.^{[8]} Es konnte jedoch keine katalysierte Kreuzkupplung von Arylzink-Reagenzien mit Arylhalogeniden unter verschiedenen Reaktionsbedingungen beobachtet werden.

Die Aufgabe der vorliegenden Erfindung ist folglich, ein einfaches Verfahren zur gezielten Knüpfung von Kohlenstoff-Kohlenstoff-Bindungen zwischen Arylen, Alkenen und Alkinen in hohen Ausbeuten und zu geringen Kosten bereitzustellen.

Diese Aufgabe wird erfindungsgemäß durch den Anspruch 1 gelöst. Bevorzugte Ausführungsformen sind in den abhängigen Ansprüchen dargelegt.

### Zusammenfassung der Erfindung

Die Erfinder wandten ihre Aufmerksamkeit anderen organometallischen Verbindungsklassen zu und fanden heraus, dass Organokupfer-Verbindungen^{[9]} des Typs **1,** mit verschiedenen funktionalisierten Arylhalogeniden (3) in Gegenwart katalytischer Mengen von Fe oder Co in einem polaren Lösungsmittel oder Lösungsmittelgemisch reagieren und zu polyfunktionalen Biarylen des Typs **4** (Tabelle 1 and 2) führen.

R¹-Ar¹-Ar²-R² (**4**)

Organokupfer-Verbindungen des Typs **1** können durch die Reaktion funktionalisierter Arylmagnesiumhalogenide (**2**)^{[10]} mit CuCN 2LiCl^{[11]} dargestellt werden. Der Begriff Aryl soll hier und im Folgenden als Aryl, Heteroaryl, Alken, oder Alkin verstanden werden. Diese Verbindungen können einfach oder mehrfach substituiert sein. Wesentlich für die Erfindung ist das Vorliegen einer Aryl-, Alken- oder Alkinverbindung, an deren charakteristischen Aryl-, Alken- oder Alkinmerkmalen die Reaktionsführung ansetzt.

Ein erster Aspekt der vorliegenden Erfindung betrifft ein Verfahren zur Herstellung einer Verbindung der allg. Formel **4**

R¹-Ar¹-Ar²-R² **(4)**

indem eine Verbindung der allg. Formel **(1)**

R¹-Ar¹-CuZMgY **(1)**

oder eine Verbindung der allg. Formel **(5)**

R¹-Ar¹-CuZLi **(5)**

mit einer Verbindung der allg. Formel **(3)**

R²-Ar²-X **(3)**

unter Einwirkung eines Co- oder Fe-Katalysators in einem Lösungsmittel umgesetzt wird,
wobei
X eine für eine nukleophilen Substitution geeignete Abgangsgruppe;
Y Cl, Br, I;
Z CN, Cl, Br, I, SCN, NR¹R², SR¹, PR¹R², Alkyl, Alkinyl;
R¹ und R² unabhängig voneinander ein oder mehrere Substituenten aus H;
substituiertem oder unsubstituiertem Aryl oder Heteroaryl, das ein oder mehrere Heteroatome enthält; geradkettigem, verzweigtem oder cyclischen, substituierten oder unsubstituierten Alkyl, Alkenyl, Alkinyl; oder Derivaten davon;
Ar¹ und Ar² unabhängig voneinander ein Aryl, kondensiertes Aryl, Heteroaryl oder kondensiertes Heteroaryl, das ein oder mehrere Heteroatome enthält; ein Alkenyl oder
Alkinyl; oder Derivate davon
sein können.

Die Abgangsgruppe X stellt eine für eine nukleophile Substitution üblicherweise verwendete Abgangsgruppe dar. Die mit Ar bezeichnete Gruppe kann auch mit mehreren der Definition von R genügenden Substituenten, sofern möglich, substituiert sein.

Gemäß einer Ausführungsform der Erfindung wird die Umsetzung bei einer Temperatur zwischen 0°C und 150°C, bevorzugt zwischen 10°C und 120°C, noch bevorzugter zwischen 20°C und 100°C, am bevorzugtesten zwischen 25°C und 80°C durchgeführt.

Gemäß einer anderen Ausführungsform umfasst der Katalysator einen Fe(III)-Komplex, ein Fe(III)-Salz, einen Fe(II)-Komplex, ein Fe(II)-Salz, oder eine reduzierte Form eines Fe-Salzes oder -Komplexes, bevorzugt Fe(acac)₃, mit acac = Acetylacetonat.

Als Eisenverbindungen können beliebige Eisen(II)- oder Eisen(III)-Salze oder -Komplexe wie z.B. FeCl₂, FeCl₃, FeBr₂, FeBr₃, Fe(OAc)₂, Fe(OAc)₃, usw. und andere Eisen-Komplexe mit Eisen in anderen Oxidationsstufen, auch reduzierte Eisenkomplexe, in denen das Eisen eine negative Oxidationsstufe besitzt, eingesetzt werden.

Gemäß einer weiteren anderen Ausführungsform umfasst der Katalysator einen Co(II)- oder Co(III)-Katalysator.

Gemäß einer anderen Ausführungsform wird der Katalysator aus der Gruppe, die CoCl₂, CoBr₂, Co(OAc)₂, Co(Bzac)₂, CoBr₂dppe, Co(acac)₂ und Co(acac)₃ umfasst, ausgewählt und bevorzugt Co(acac)₂ verwendet, wobei OAc für Acetoxy, Bzac für Benzoylacetonat und dppe für 1,2-Bis(diphenylphosphino)ethan stehen.

Als Kobaltverbindungen können Kobalt-Salze oder -Komplexe mit beliebiger Oxidationsstufe des Kobalts verwendet werden, auch Kobalt-Komplexe, in denen das Kobalt eine negative Oxidationsstufe besitzt.

Gemäß einer anderen Ausführungsform wird(werden) zusätzlich Ethen und/oder ein oder mehrere Ethenderivat(e), bevorzugt elektronenarme Ethenderivate, besonders bevorzugt Maleinsäureanhydrid, Tetracyanoethylen, Styrol oder ein Styrolderivat, noch bevorzugter ein elektronenarmes Styrolderivat, und am bevorzugtesten 4-Fluorstyrol, während der katalytischen Umsetzung zugegeben.

Gemäß einer anderen Ausführungsform wird das Ethen oder Ethenderivat in einer Menge von 0 - 50 Mol%, bevorzugt 1 - 30 Mol%, besonders bevorzugt 5 - 25 Mol%, am bevorzugtesten 10 - 20 Mol%, bezogen auf die molare Menge der Verbindung **(3)** zugegeben.

Gemäß einer anderen Ausführungsform werden zusätzlich ein oder mehrere Salze, bevorzugt Tetrabutylammoniumchlorid, Tetrabutylammoniumbromid, Kaliumiodid, Lithiumiodid und/oder am bevorzugtesten Tetrabutylammoniumiodid während der katalytischen Umsetzung zugegeben.

Gemäß einer anderen Ausführungsform kann X bevorzugt F, Cl, Br, I, OTf, OTs, N₂⁺, bevorzugter Cl oder Br, noch bevorzugter I sein.

Gemäß einer anderen Ausführungsform kann X bevorzugt F, Cl, Br, I, OTf, OTs, N₂⁺, bevorzugter F, Cl, I oder OTs, noch bevorzugter Br sein.

Gemäß einer weiteren Ausführungsform wird als Lösungsmittel ein polares Lösungsmittel oder Lösungsmittelgemisch, bevorzugt ein etherisches Lösungsmittel oder Lösungsmittelgemisch, und am bevorzugtesten ein Lösungsmittel oder Lösungsmittelgemisch, ausgewählt aus der Gruppe, die Tetrahydrofuran (THF), Dimethoxyethan (DME), N-Methylpyrrolidon (NMP), N,N'-Dimethylpropylenharnstoff (DMPU) und N,N-Dimethylacetamid (DMAC) umfasst, verwendet.

Gemäß einer anderen Ausführungsform wird die Verbindung **(1)** oder **(5)** in einem molaren Verhältnis von 0,9 - 5, bevorzugt in einem molaren Verhältnis von 1 - 3, noch bevorzugter in einem molaren Verhältnis von 1,2 - 2,5 bezogen auf die molare Menge an Verbindung **(3)** zugegeben.

Gemäß einer anderen Ausführungsform ist Z bevorzugt CN.

Gemäß einer weiteren Ausführungsform können R¹ und R² unabhängig voneinander ein oder mehrere substituierte oder unsubstituierte C₄-C₂₄ Aryle oder C₃-C₂₄ Heteroaryle, die ein oder mehrere Heteroatome wie B, O, N, S, Se, P enthalten; geradkettige oder verzweigte, substituierte oder unsubstituierte C₁-C₂₀ Alkyle, C₁-C₂₀ Alkenyl, C₁-C₂₀ Alkinyle; oder substituierte oder unsubstituierte C₃-C₂₀ Cycloalkyle; oder Derivate davon sein.

Diese neue Vorgehensweise eröffnet einen wirtschaftlichen Zugang (ca. drei mal billiger verglichen mit Pd-katalysierten Reaktionen) zur Durchführung von Aryl-Aryl-Kreuzkupplungen.

### Ausführliche Beschreibung der Erfindung

Die vorliegende Erfindung wird jetzt mit Verweis auf die Figuren ausführlicher beschrieben.

Wenn nicht anders festgelegt, sollen die technischen und wissenschaftlichen Begriffe, die hierin verwendet werden, die gleiche Bedeutung besitzen, wie sie von einem Fachmann auf dem Gebiet dieser Erfindung verstanden werden.

### Eisen-katalysierte Reaktionsführung

Organokupfer-Verbindungen^{[9]} des Typs **1**, welche durch die Reaktion funktionalisierter Arylmagnesiumchloride (**2**)^{[10]} mit CuCN 2LiCl^{[11]} dargestellt werden, können mit verschiedenen funktionalisierten Arylhalogeniden (3) in Gegenwart katalytischer Mengen von Fe(acac)₃ (10 mol%) in einem DME:THF-Gemisch (3:2) umgesetzt werden und zu polyfunktionalen Biphenylen des Typs **4** (Tabelle 1 and 2) führen:

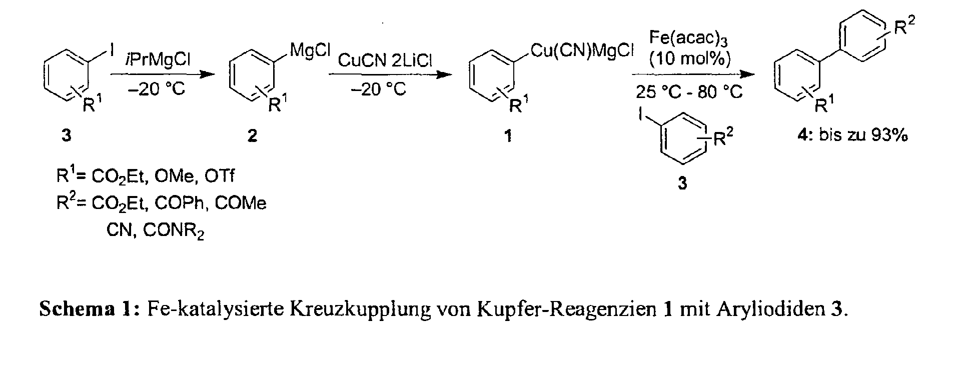

Die Reaktion läuft zwischen 25°C und 80°C ab. Bei der Verwendung von Organokupfer-Reagenzien (**1**) ist das Ausmaß der Homokupplung bemerkenswerterweise verringert und die Kreuzkupplung erfolgt bereitwillig. Die Art der Abgangsgruppe des aromatischen Elektrophils **3** ist ebenfalls wichtig (Tabelle 1). Deshalb ist die Reaktion von PhCu(CN)MgCl (**1a**) mit 2-Iodbenzophenon **(3a)** innerhalb von 30 min bei 25 °C vollständig. Ohne Fe(acac)₃ beobachtet man weniger als 5% des Biphenyls **4a** nach 30 min und nach 48 h Reaktionszeit kann nur ein Umsatz von ca. 54% beobachtet werden (Eintrag 1 in Tabelle 1). Das entsprechende Bromid (2-Brombenzophenon; Eintrag 2) reagiert ebenfalls schnell, führt aber nach 30 min nur zu einem Umsatz von 86%. Eine Reaktionszeit von 18 h verbessert den Umsatz nur geringfügig (93%). In ähnlicher Weise führt auch das 2-Chlorbenzophenon nicht zu vollständigem Umsatz (75% nach 30 min, aber nur 77% nach 18 h). Ein signifikant langsamerer Umsatz kann mit einem Triflat-Substituenten (X=OTf) beobachtet werden. Die Tatsache, dass 2-Chlorbenzophenon reagiert, deutet darauf hin, dass der Mechanismus der Reaktion nicht mit einer Halogen-Kupfer-Austauschreaktion einhergeht. In Abwesenheit des Eisen-Katalysators konnte keine Reaktion für 2-Chlorbenzophenon beobachtet werden.

Interessanterweise haben die Erfinder bei der Umsetzung von Arylkupfer-Verbindungen mit Arylhalogeniden eine umgekehrte Reaktivität als bei Fe(III)-katalysierten Reaktionen mit Alkylmagnesium-Verbindungen beobachtet, für welche Aryliodide weniger gute Substrate darstellen als Arylbromide oder -chloride.^{[7]} Die Erfinder haben dann den Anwendungsbereich der Reaktion untersucht und eine bemerkenswerte Chemoselektivität und Kompatibilität mit funktionellen Gruppen festgestellt. Die Reaktion von PhCu(CN)MgCl (**1a**) mit 2-Iodbenzophenon **(3a)** ist besonders schnell (30 min) und das entsprechende Keton **4a** kann in 93% Ausbeute isoliert werden (Eintrag 1 in Tabelle 2). 4-Iodbenzophenon **(3b)** reagiert bei 25 °C innerhalb von 4h und führt zum gewünschten Keton **4b** in 80% Ausbeute (Eintrag 2). Bemerkenswerterweise geht ein Methylketon wie 2-Iodphenylmethylketon **(3c)** die Eisen-katalysierte Kreuzkupplung ohne jegliche konkurrierende Deprotonierung des Methylketons oder eine Addition an die Carbonylfunktion ein. Funktionalisierte Arylmagnesium-Reagenzien, die eine Estergruppe in *ortho-* oder *para*-Position tragen wie **1b**, **1c** oder **1d**, gehen ebenfalls innerhalb weniger Stunden die Kreuzkupplung ein und führen zu den erwarteten Produkten **4d-f** in 68-86% Ausbeute (Einträge 4-6). Das sterisch gehinderte funktionalisierte 2,2'-substitutierte Biphenyl **4d** wird so in 75% Ausbeute dargestellt (Eintrag 4). Auch Grignard-Reagenzien, die eine Donor-Gruppe besitzen wie **1e**, reagieren gut und ergeben das Keton **4g** in 76% Ausbeute (Eintrag 7). Verschiedene *para*-substituierte Aryliodide, die eine elektronenziehende Gruppe tragen, wie ein Cyanid (3e), ein Amid (**3f**) oder ein Keton (3b) reagieren in einer milden Kreuzkupplung und führen zu den Produkten **4h-j** in 50-70% Ausbeute (Einträge 8-10). Erstaunlicherweise reagiert das Kupfer-Reagenz **1f**, welches eine Triflat-Gruppe besitzt, mit Ethyl-2-iodbenzoat **3g** zum Biphenyl **4k** in 62% Ausbeute (Eintrag 11). Die Erfinder haben festgestellt, dass die Gegenwart eines -elektronenziehenden Substituenten am Aryliodid die Kreuzkupplung beschleunigt und Donor-Substituenten die Reaktion erheblich verlangsamen. Deshalb führt 4-Iodanisol nur zu geringem Umsatz bei 80 °C nach 12 h. Die Erfinder haben ebenfalls die Reaktionsgeschwindigkeit der Kreuzkupplung für 2-, 3- und 4-substituiertes Ethyliodbenzoat (**3g-i**) mit PhCu(CN)MgCl in der Gegenwart von Fe(acac)₃ (10 mol%) miteinander verglichen (Schema 2). Interessanterweise reagieren das *ortho-* und *para*-substituierte Iodbenzoat **3g** und **3i** sehr viel schneller im Gegensatz zu dem *meta*-substituierten Ester **3h,** welcher eine fünf mal so lange Reaktionszeit bis zum vollständigen Umsatz benötigt. Dies kann darauf hindeuten, dass der langsamste Schritt der Kreuzkupplung der nukleophile Angriff der katalytisch aktiven Spezies am Benzoat **3g-i** ist. Die Erfinder der vorliegenden Erfindung haben mit den substituierten Iodestern **3g-i** ebenfalls die Pd- und Ni-katalysierte Kreuzkupplung durchgeführt und herausgefunden, dass im Falle der Pd-Katalyse, der *para-*Iodester 3i bedeutend schneller reagiert als **3g** and **3h,** wohingegen unter den Bedingungen der Ni-Katalyse der *meta*-Iodester **3h** am schnellsten reagiert. Diese Ergebnisse legen nahe, dass die Mechanismen der Ni-, Pd- und Fe-katalysierten Kreuzkupplungsreaktionen sehr verschieden sind, da die elektronischen und sterischen Effekte der Substituenten diese in verschiedener Weise beeintlussen.

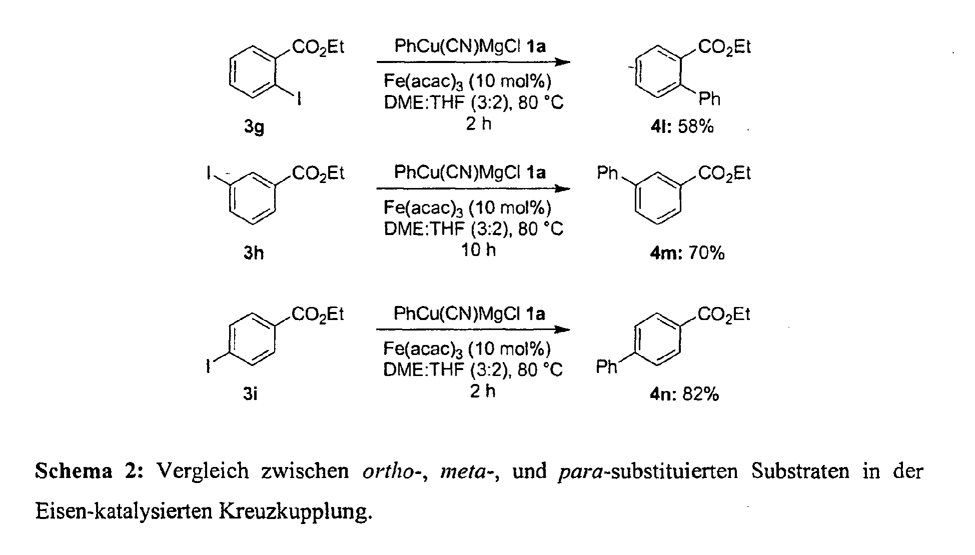

Heterozyklische Verbindungen erweisen sich ebenfalls als geeignet für diese Kreuzkupplungsreaktion. Demnach reagieren die Kupfer-Reagenzien **1g** und **1h** unter StandardBedingungen mit den Iodiden **3f** und **3e** und führen zu dem funktionalisierten Indol **4o** und dem Pyridin **4p** in jeweils 85% und 57% Ausbeute (Schema 3).

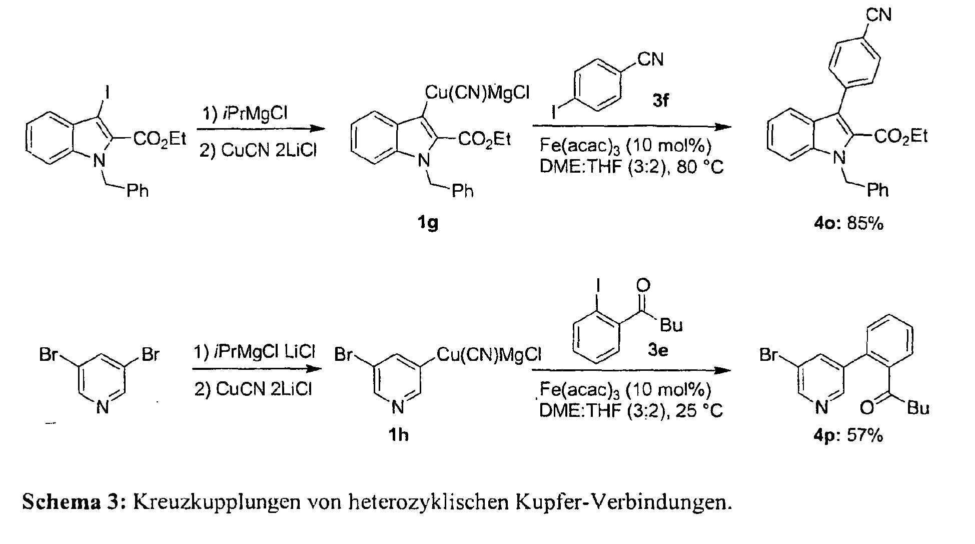

Die Eisen-katalysierte Kreuzkupplung von Heteroaryl- und Arylkupfer-Verbindungen, welche aus den entsprechenden Organomagnesium-Verbindungen dargestellt werden, läuft bereitwillig mit funktionalisierten Aryliodiden ab. Die elektronenarmen elektrophilen Iodide gehen dabei leichter die Kreuzkupplungsreaktion ein.

### Kobalt-katalysierte Reaktionsführung

Als Katalysator kann, wie die folgenden Beispiele zeigen, auch ein Co-Salz eingesetzt werden.

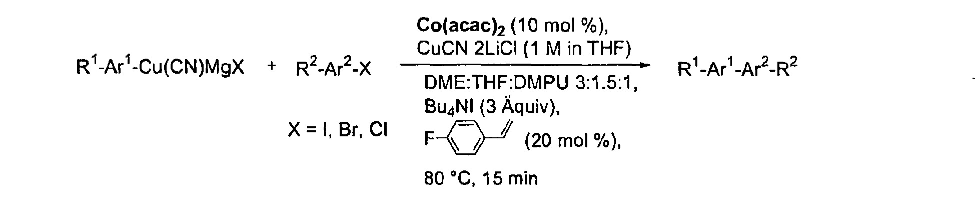

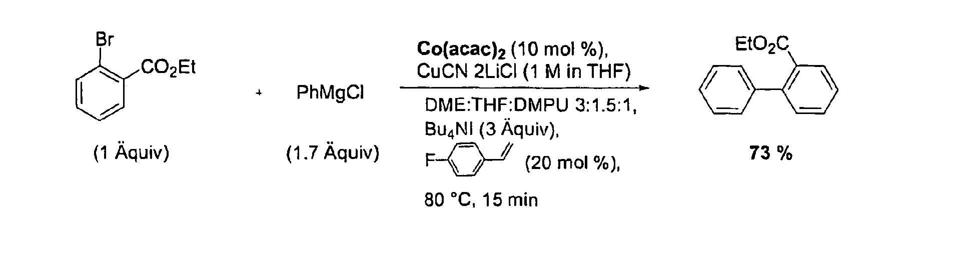

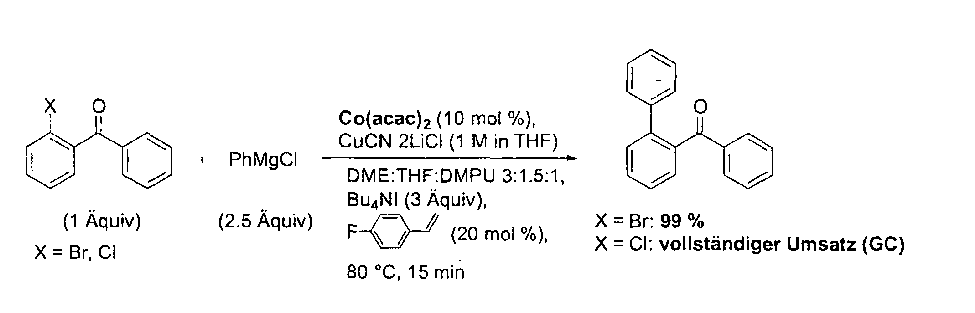

Ein wichtiger Schlüsselschritt zur Aryl-Aryl-Kupplung zwischen funktionalisierten Aryl- oder Heteroarylmagnesiumverbindungen mit funktionalisierten Aryl- oder Heteroarylhalogeniden ist dabei die Transmetallierung der Arylmagnesiumverbindung (R¹-ArMgCl) auf Kupfer um so Kupferorganyle des Typs R¹-ArCu(CN)MgX zu erhalten. Neben des Kupfersalzes CuCN sind auch die Salze CuCl, CuBr, Cul, CuSCN usw. als Kupferquelle zur Transmetallierung verwendbar.

Die Reaktion findet in einem Lösungsmittelgemisch aus THF, DME und einem polaren Cosolvens statt. Als solches kommen NMP, DMPU aber auch DMAC zum Einsatz.

Weitere wichtige Bestandteile der Reaktion sind die Zugabe eines Salzes wie Tetrabutylammoniumiodid und eines elektronenarmen Styrolderivates wie 4-Fluorstyrol. Neben Tetrabutylammoniumiodid können auch Tetrabutylammoniumbromid, Tetrabutylammoniumchlorid, Kaliumiodid oder Litiumiodid verwendet werden.

Der Katalysator stellt ein Kobaltsalz dar. Als bestes hat sich Co(acac)₂ erwiesen. Die Reaktion wird aber auch von CoCl₂, CoBr₂, Co(OAc)₂, Co(Bzac)₂, CoBr₂dppe oder Co(acac)₃ katalysiert.

Die Kupplungspartner für die funktionalisierten Organokupferverbindungen sind funktionalisierte Arylhalogenide. Dabei können Iodide, Bromide und teilweise Chloride eingesetzt werden. Die wichtigsten sind dabei die Bromide und Chloride, da diese in der Regel billiger und damit für die Industrie interessanter sind als Iodide.

Mit der in dieser Erfindungsmeldung bereits beschriebenen Methode für Kreuzkupplungen zwischen Arylbromiden und -chloriden und Arylkupfer- bzw. Arylmagnesiumverbindungen können auch Arylfluoride und -tosylate (OTs) umgesetzt werden. Allerdings sind dafür 3 Äquivalente an Arylkupferverbindung nötig.

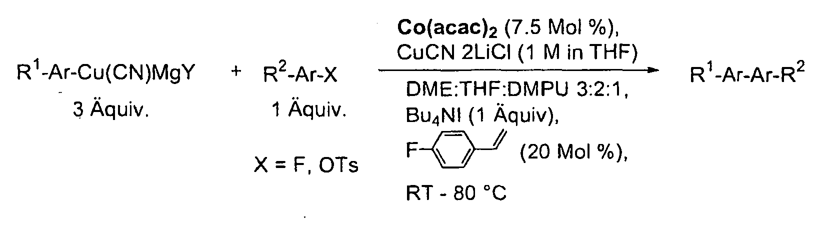

Beim Einsatz von 2,3,4,5,6-Pentafluorbenzophenon **5h** können gleichzeitig zwei Kreuzkupplungen durchgeführt werden. Entsprechend sind dabei 6 Äquivalente an Kupferreagens notwendig, wie aus Schema 4 ersichtlich ist.

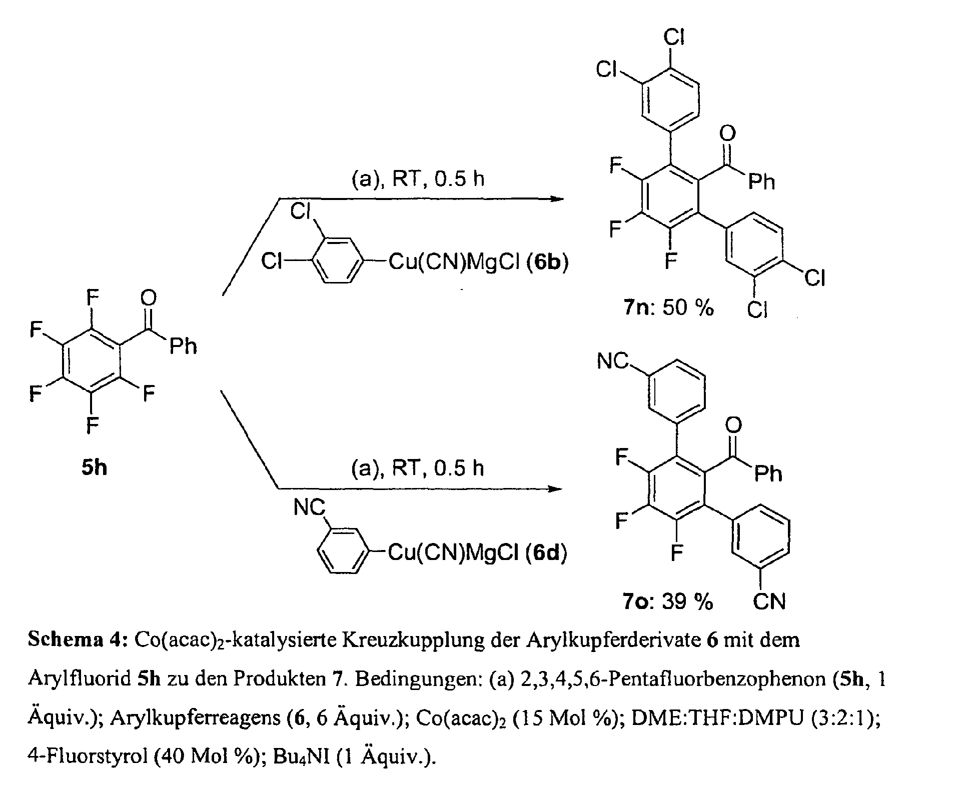

Der Einsatz der polaren Cosolventien, der Salze und des Styrolderivates und des Kobaltsalzes ist unabdingbar, um die Kupplungsreaktion mit Bromiden und Chloriden vollständig und schnell ablaufen zu lassen. Ohne diese Zusätze findet keine vollständige Reaktion statt oder sie benötigt Tage anstatt weniger Minuten oder Stunden.

### Beispiel 1:

### Synthese von 4'-Cyanobiphenyl-4-carbonsäureethylester (4h):

In einem 25 mL Schlenkrohr, mit magnetischem Rührkern und Septum, wird Ethyl-4-iodbenzoat (855mg, 3.10 mmol) vorgelegt, DME (5 mL) zugegeben und die Lösung auf -20 °C gekühlt. Anschließend wird *i*PrMgCl (3.3 mL, 3.0 mmol, 0.90 M in THF) zugegeben. Die Reaktionsmischung wird 15 min bei dieser Temperatur gerührt und anschließend mit einer CuCN 2LiCl Lösung (2.8 mL, 2.8 mmol, 1 M in THF) versetzt. Die Reaktionsmischung wird für weitere 10 min gerührt. Eine Lösung von 4-Iodbenzonitril (229 mg, 1.00 mmol) und Fe(acac)₃ (35 mg, 0.10 mmol) gelöst in DME (3 mL) wird auf einmal zugegeben und die Reaktionsmischung dann für 3 h auf 80 °C erhitzt. Die Reaktion wird durch Zugabe von ges. NH₄Cl_{(aq.)} beendet und mit CH₂Cl₂ (3x40 mL) extrahiert. Die vereinigten organischen Phasen werden mit ges. NH₄Cl_{(aq.)}/NH₃ (9:1) (50 mL) und ges. NaCl_{(aq.)} (50 mL) gewaschen, über Na₂SO₄ getrocknet, abfiltriert und das Lösungsmittel unter vermindertem Druck abdestilliert. Die säulenchromatographische Reinigung (Pentan/Diethylether = 9:1) ergab **4h** als einen farblosen Feststoff (181 mg, 72%).

### Beispiel 2:

### Reaktionsgeschwindigkeiten bei unterschiedlichen Abgangsgruppen X

Entsprechend der Versuchsvorschrift von Beispiel 1 wurden Umsetzungen mit unterschiedlich substituierten Benzophenonen durchgeführt. Die entsprechenden Substituenten an 2-Position sind in Tabelle 1 aufgeführt, ebenso wie die dabei erzielten Umsätze.

**Tabelle 1: Umsatzgeschwindigkeit von 2-substituierten Benzophenonen mit PhCu(CN)MgCl in Gegenwart von Fe(acac)₃.**

| | | |
|---|---|---|
| 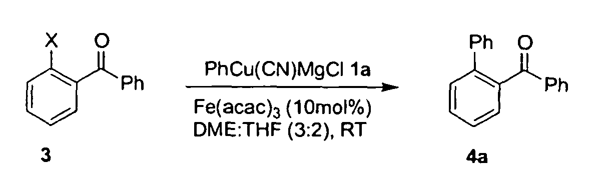 | | |

| Eintrag | **X** | Umsatz (%)^{[a]} |
|---|---|---|
| 1 | I | 100, (<5)^{[b]}, (55)^{[c]} |
| 2 | Br | 86, (93)^{[d]} |
| 3 | Cl | 75, (77)^{[d]} |
| 4 | OTf | 35, (100)^{[e]} |
| 5 | OTs | 0 |

| | | |
|---|---|---|
| ^{[a]} Umsatz nach 30 min Reaktionszeit, bestimmt durch GC-Analyse von Reaktionsproben; ^{[b]} Umsatz in der Abwesenheit von Fe(acac)₃ nach 30 min; ^{[c]} Umsatz nach 48 h in der Abwesenheit von Fe(acac)₃; ^{[d]} Umsatz nach 18 h Reaktionszeit; ^{[e]} Umsatz nach 2 h Reaktionszeit in THF. | | |

### Beispiel 3:

### Umsetzungen unter Fe-Katalyse

Entsprechend der Versuchsvorschrift in Beispiel 1 wurden alle in Tabelle 2 dargestellten Verbindungen synthetisiert. Dabei sind die entsprechenden Edukte **1** und **3,** wie sie in der Tabelle dargestellt sind, zu den entsprechenden Produkten **4** umgesetzt worden. Die Ausbeute der Reaktion ist in der letzten Spalte der Tabelle angegeben.

**Tabelle 2: Produkte des Typs 4, die durch Fe-katalysierte Kreuzkupplung von funktionalisierten Arylkupfer-Verbindungen (1) mit Aryliodiden erhalten werden.**

| Eintrag | Arylkuprat **1**^{[a]} | Aryliodid **3** | Produkt des Typs **4** | Ausbeute (%)^{[b]} |
|---|---|---|---|---|
| 1 | PhCu **1a** | 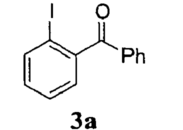 | 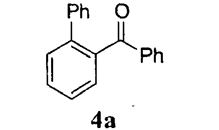 | 93 |
| 2 | PhCu **1a** | 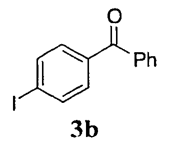 | 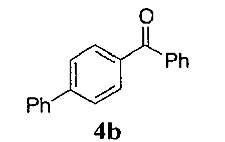 | 80 |
| 3 | PhCu **1a** | 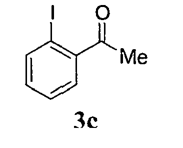 | 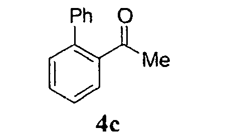 | 86 |
| 4 | 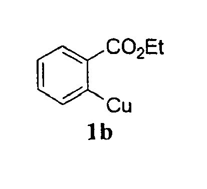 | 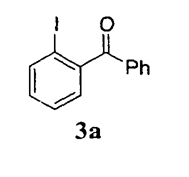 | 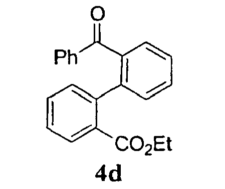 | 75 |
| 5 | 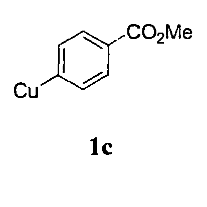 | 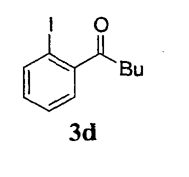 | 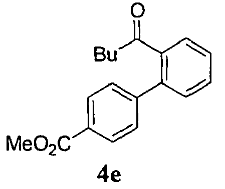 | 68 |
| 6 | 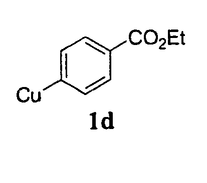 | 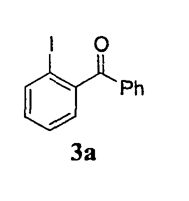 | 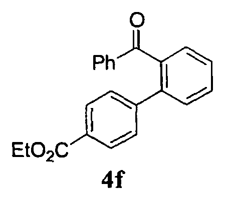 | 86 |
| 7 | 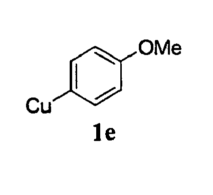 | 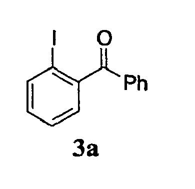 | 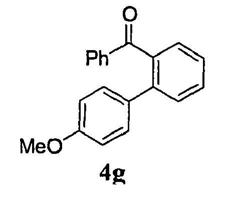 | 76 |
| 8 | 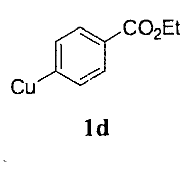 | 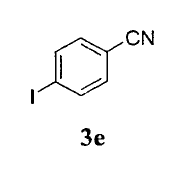 | 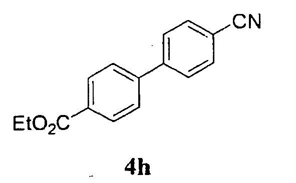 | 72 |
| 9 | 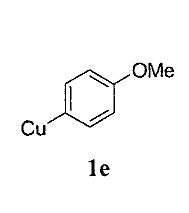 | 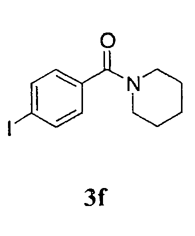 | 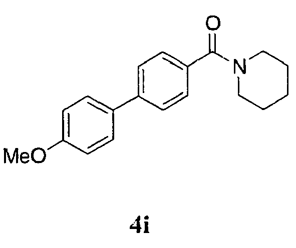 | 58 |
| 10 | 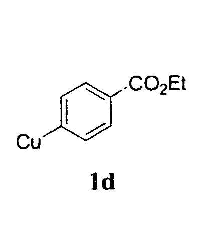 | 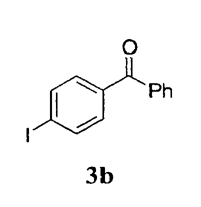 | 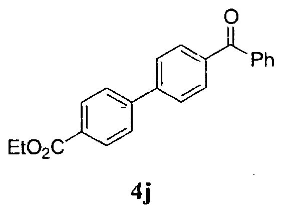 | 50 |
| 11 | 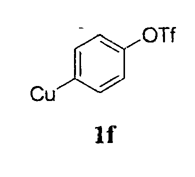 | 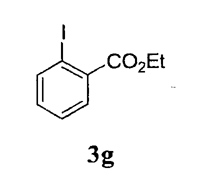 | 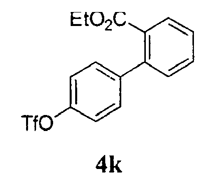 | 62 |

| | | | | |
|---|---|---|---|---|
| [a] Das Kupfer-Reagenz wird besser durch ArCu(CN)MgCl dargestellt. [b] Ausbeute des analytisch reinen Produkts. | | | | |

### Beispiel 4:

### Synthese von 1,1'-Biphenyl-2-carbonsäureethylester:

In einem 25 mL Schlenkkolben, mit magnetischem Rührkern und Septum, wird DME (5.0 mL) vorgelegt, PhMgCl (1.42 mL, 1.70 mmol, 1.2 M in THF) und CuCN 2LiCl Lösung (1.90 mL, 1.90 mmol, 1 M in THF) zugegeben und die Lösung bei Raumtemperatur ca. 10 min gerührt. Anschließend werden DMPU (2.0 mL), Tetrabutylammoniumiodid (1.11 g, 3.00 mmol), 4-Fluorstyrol (25 mg, 0.21 mmol), 2-Brombenzoesäureethylester (229 mg, 1.00 mmol) und eine Lösung von Co(acac)₂ (25.8 mg, 0.10 mmol) in DME (1.0 mL) zugegeben. Die entstandene Suspension wird 15 Minuten auf 80 °C erhitzt. Das Reaktionsgemisch wird mit ges. NH₄Cl_{(aq.)}/NH₃ (4:1) (2x50 mL) extrahiert, die vereinigten wässrigen Phasen mit Diethylether (3x50 mL) extrahiert und die vereinigten organischen Phasen mit ges. NaCl_{(aq.)} (50 mL) gewaschen. Anschließend wird über Na₂SO₄ getrocknet, abfiltriert und das Lösungsmittel unter vermindertem Druck abdestilliert. Die säulenchromatographische Reinigung (Pentan/Diethylether = 19:1) ergab das Produkt als eine farblosen Flüssigkeit (165 mg, 73 %).

### Beispiel 5:

### Synthese von 2'-(4-Methoxybenzoyl)-biphenyl-4-carbonitril (7g):

In einem mit Rührfisch und Septum versehenen 25 mL Schlenk-Kolben wurde *i*PrMgCl LiCl (2.63 mL, 3.15 mmol, 1.2 M in THF) gefüllt. Die Lösung wurde auf -20 °C gekühlt und 4-Brombcnzonitril (544 mg, 2.99 mmol) wurde zugegeben. Danach wurde die Reaktionsmischung auf 0 °C erwärmt und 2 h bei dieser Temperatur gerührt. Nun wurde CuCN 2LiCl-Lösung (3.2 mL, 3.2 mmol, 1 M in THF) zugegeben. Nach 10 min wurden DME (6.0 mL), DMPU (2.0 mL), Bu₄NI (370 mg, 1.00 mmol), 4-Fluorstyrol (25 mg, 0.20 mmol), Co(acac)₂ (19.3 mg, 0.075 mmol) und (2-Fluorphenyl)(4-methoxyphenyl)methanon (230 mg, 1.00 mmol) zugegeben. Die Reaktionsmischung wurde 0.25 h bei Raumtemperatur gerührt und anschließend mit ges. NH₄Cl_{(aq.)}/NH₃-Mischung (9:1) (50 mL) gequencht. Die organische Phase wurde ein zweites Mal mit ges. NH₄Cl_{(aq.)}/NH₃-Mischung (9:1) (50 mL) gewaschen und die vereinigten wässrigen Phasen wurden mit EtOAc (3x40 mL) extrahiert. Die vereinigten organischen Phasen wurden mit ges. NaCl-Lösung (50 mL) gewaschen, über MgSO₄ getrocknet, vom Trocknungsmittel abfiltriert und das Lösungsmittel im Vakuum eingeengt. Die säulenchromatographische Reinigung auf Kieselgel
(Pentan/Diethylether = 4:1) lieferte 7g als einen farblosen Feststoff (271 mg, 0.87 mmol, 87 %, Schmp.: 120.8 - 122.9 °C).

### Beispiel 6:

### Umsetzung unter Co-Katalyse

Gemäß obiger Arbeitsvorschrift in Beispiel 5 lassen sich alle in Tabelle 3 angegebenen Produkte **7** darstellen. Dabei müssen in der Arbeitsvorschrift aus Beispiel 5 die entsprechenden Edukte **5** und **6** sowie die Bedingungen gemäß der Tabelle ersetzt werden. Die Ausbeute ist in der rechten Spalte der Tabelle 3 angegeben.

**Tabelle 3: Durch Co(acac)₂-katalysierte Kreuzkupplung der Arylkupferderivate 6 mit den Arylfluoriden 5 zu den Produkten 7.**

| Eintrag | Aryltluorid **5** | Arylkupferreagens **6**^{[a]} | Produkt **7** | Bedingungen [°C, h] | Ausb. [%]^{[b]} |
|---|---|---|---|---|---|
| | 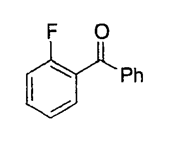 | 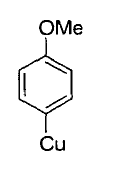 | 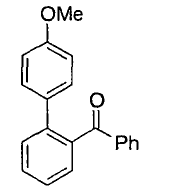 | | |
| 1 | **5a** | **6a** | **7a** | 25, 2 | 98 |
| | 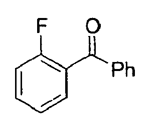 | 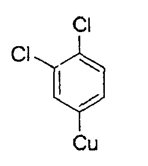 | 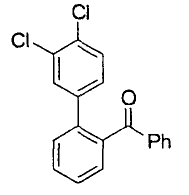 | | |
| 2 | **5a** | **6b** | **7b** | 25, 0.5 | 87 |
| | 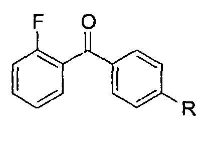 | 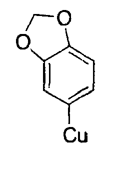 | 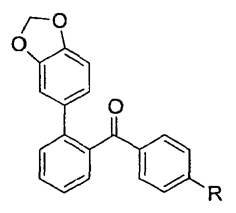 | | |
| 3 | **5b:** R = OPiv | **6c** | 7c: R = OPiv | 25, 16 | 72 |
| 4 | **5c:** R = OMe | **6c** | 7d: R = OMe | 25, 3 | 98 |
| 5 | 5d: R = NMe₂ | **6c** | 7e: R = NMe₂ | 25,3 | 95 |
| | 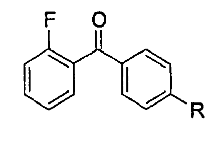 | 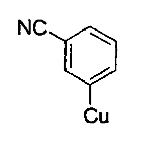 | 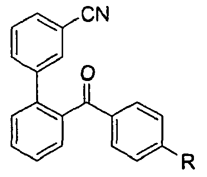 | | |
| 6 | **5b**: R = OPiv | **6d** | **7f**: R = OPiv | 25, 0.25 | 52 |
| | 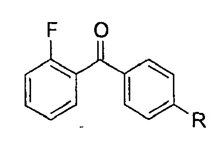 | 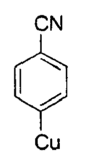 | 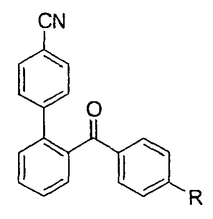 | | |
| 7 | **5c:** R = OMe | **6e** | **7g:** R = OMe | 25, 0.25 | 87 |
| | 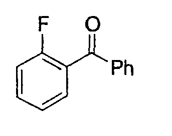 | 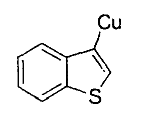 | 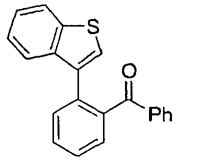 | | |
| 8 | **5a** | **6f** | **7h** | 80, 1 | 94 |
| | 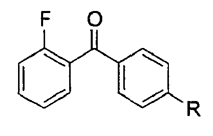 | 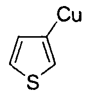 | 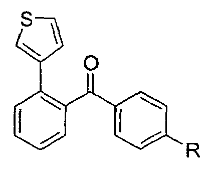 | | |
| 9 | **5d:** R = NMe₂ | **6g** | **7i:** R = NMe₂ | 80, 1 | 42 |
| | 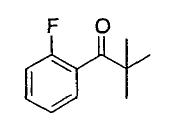 | 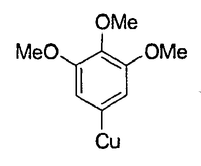 | 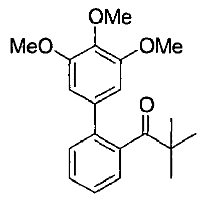 | | |
| 10 | **5e** | **6h** | **7j** | 80, 1 | 71 |
| | 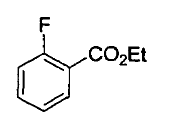 | 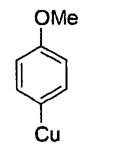 | 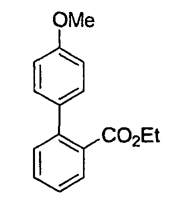 | | |
| 11 | **5f** | **6a** | **7k** | 80,0.25 | 51 |
| | 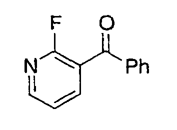 | 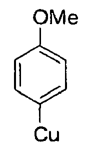 | 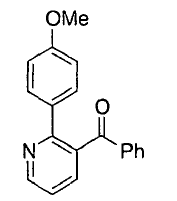 | | |
| 12 | **5g** | **6a** | **7l** | 80, 1 | 25 |
| | 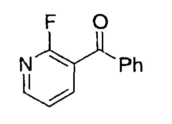 | 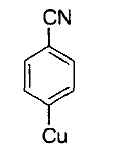 | 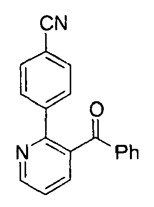 | | |
| 13 | **5g** | **6e** | **7m** | 80, 3 h | 15 |

| | | | | | |
|---|---|---|---|---|---|
| [a] Das Kupferreagens wird besser repräsentiert durch ArCu(CN)MgCl. [b] Ausbeute an analytisch reinem Produkt. | | | | | |

### Beispiel 7:

### Synthese von (E)-1-(Hex-1-enyl)-4-methoxybenzene

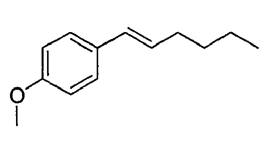

In einem 25 mL Schlenkrohr, mit magnetischem Rührkern und Septum, wird 4-Methoxyphenyl-Grignard (3.75 mL, 3.00 mmol, 0.8 M in THF) in DME (5 mL) vorgelegt, die Lösung auf -20 °C gekühlt und anschließend mit einer CuCN 2LiCl Lösung (2.8 mL, 2.8 mmol, 1 M in THF) versetzt. Die Reaktionsmischung wird für 10 min gerührt. Anschließend wird eine Lösung von 1-Iodhexen (210 mg, 1.00 mmol) und Fe(acac)₃ (35 mg, 0.10 mmol) gelöst in DME (3 mL) auf einmal zugegeben und die Reaktionsmischung dann für 3 h auf 80 °C erhitzt. Die Reaktion wird durch Zugabe von ges. NH₄Cl_{(aq.)} beendet und mit CH₂Cl₂ (3x40 mL) extrahiert. Die vereinigten organischen Phasen werden mit ges. NH₄Cl_{(aq.)}/NH₃ (9:1) (50 mL) und ges. NaCl_{(aq.)} (50 mL) gewaschen, über Na₂SO₄ getrocknet, abfiltriert und das Lösungsmittel unter vermindertem Druck abdestilliert. Die säulenchromatographische Reinigung (Pentan/Diethylether = 19:1) ergab (*E*)-1-(Hex-1-enyl)-4-methoxybenzene als einen farbloses Öl (118 mg, 62%).
**¹H-NMR** (300 MHz, CDCl₃, 25 °C): δ = 7.19 (d, *J*= 8.7 Hz, 2H), 6.75 (d, *J*= 8.7 Hz, 2H), 6.24 (d, *J*= 15.4 Hz, 1H), 6.04-5.94 (m. 1H), 3.70 (s, 3H), 2.00 (q, *J*= 6.8 Hz, 2H), 1.41-1.24 (m, 5H), 0.84 (t, *J*= 7.7 Hz, 3H).
**¹³C-NMR** (75 MHz, CDCl₃, 25 °C): δ = 158.58, 130.82, 129.01, 126.93, 113.88, 55.23, 32.67, 31.66, 22.24, 13.93.
**MS** (70 eV, EI): *m*/*z* (%):190 (37) [M], 147 (100), 134 (10), 115 (11), 103 (5), 91 (14), 77 (4), 65(2).
**HRMS** for **C₁₃H**₁₈**O** (190,1358): gefunden: 190.1337.

### Beispiel 8:

### Synthese von (Z)-Ethyl-3-(4-methoxyphenyl)but-2-enoate

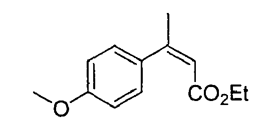

In einem 25 mL Schlenkrohr, mit magnetischem Rührkern und Septum, wird 4-Methoxyphenyl-Grignard (3.75 mL, 3.00 mmol, 0.8 M in THF) in DME (5 mL) vorgelegt, die Lösung auf -20 °C gekühlt und anschließend mit einer CuCN 2LiCl Lösung (2.8 mL, 2.8 mmol, 1 M in THF) versetzt. Die Reaktionsmischung wird für 10 min gerührt. Anschließend wird eine Lösung von (*Z*)-Ethyl-3-iodobut-2-enoate (240 mg, 1.00 mmol) und Fe(acac)₃ (35 mg, 0.10 mmol) gelöst in DME (3 mL) auf einmal zugegeben und die Reaktionsmischung dann für 3 h auf 80 °C erhitzt. Die Reaktion wird durch Zugabe von ges. NH₄Cl_{(aq.)} beendet und mit CH₂Cl₂ (3x40 mL) extrahiert. Die vereinigten organischen Phasen werden mit ges. NH₄Cl_{(aq.)}/NH₃ (9:1) (50 mL) und ges. NaCl_{(aq.)} (50 mL) gewaschen, über Na₂SO₄ getrocknet, abfiltriert und das Lösungsmittel unter vermindertem Druck abdestilliert. Die säulenchromatographische Reinigung (Pentan/Diethylether = 19:1) ergab (*Z*)-Ethyl-3-(4-methoxyphenyl)but-2-enoate als einen farbloses Öl (128 mg, 58%).
**¹H-NMR** (300 MHz, CDCl₃, 25 °C): δ = 7.35 (d, *J*= 8.6 Hz, 2H), 6.79 (d, *J*= 8.6 Hz, 2H), 6.02 (q, *J*= 1.2 Hz, 1H), 4.12 (q, *J*= 7.0 Hz, 2H), 3.75 (s, 3H), 2.46 (d, *J*= 1.3 Hz, 3H), 1.22 (t, *J*= 7.2 Hz, 3H).
**¹³C-NMR** (75 MHz, CDCl₃, 25 °C): δ = 167.01, 160.37, 154.79, 134.24, 127.57, 115.24, 113.75, 59.62, 55.21, 17.55, 14.28.
**MS** (70 eV, EI): *m*/*z* (%): 220 (95) [M], 191 (10), 175 (100), 148 (57), 132 (10), 115 (15), 91 (13), 77 (10).
**HRMS** for **C₁₃H₁₆O₃** (220,1099): gefunden: 220.1088.

### Literatur und Anmerkungen:

[1] (a) Metal catalyzed Cross-Coupling Reactions; F. Diederich; P. J. Stang Eds. Wiley-VCH, Weinheim, 1998**;** (b) J. Tsuji, Transition Metal Reagents and Catalysts: Innovations in Organic Synthesis; Wiley: Chichester, 1995**;** (c) Cross-Coupling Reactions. A Practical Guide; N. Miyaura Ed. Top. Curr. Chem. 2002, 219, Springer-Verlag Berlin, Heidelberg, New York.
[2] Handbook of organopalladium chemistry for organic synthesis, Ed. E. Negishi, Wiley-Interscience, New York, 2002**.**
[3] (a) C. Dai, G. C. Fu, J. Am. Chem. Soc. 2001, 123, 2719; (b) A. F. Littke, G. C. Fu, J. Am. Chem. Soc. 2001, 123, 6989; (c) A. F. Littke, L. Schwarz, G. C. Fu, J. Am. Chem. Soc. 2002, 124, 6343; (d) A. F. Littke, G. C. Fu, Angew. Chem. 2002, 114, 4350; Angew. Chem. Int. Ed. 2002, 41, 4176; (e) I. D. Hills, M. R. Netherton, G. C. Fu, Angew. Chem. 2003, 115, 5927; Angew. Chem. Int .Ed. 2003, 42, 5749; (g) A. C. Frisch, A. Zapf, O. Briel, B. Kayser, N. Shaikh, M. Beller, J. Mol. Catalysis A 2004, 214, 231; (h) A. Tewari, M. Hein, A. Zapf, M. Beller, Synthesis 2004, 935; (i) F. Rataboul, A. Zapf, R. Jackstell, S. Harkal, T. Riermeier, A. Monsees, U. Dingerdissen, M. Beller, Chem. Eur. J. 2004, 10, 2983.
[4] (a) M. Kumada, Pure Appl. Chem. 1980, 52, 669; (b) T.-Y. Luh, Acc. Chem. Res. 1991, 24,257; (c) S. Sengupta, M. Leite, D. S. Raslan, C. Quesnelle, V. Snieckus, J. Org. Chem. 1992, 57, 4066; (d) A. F. Indolese, Tetrahedron Lett. 1997, 38, 3513; (e) E. Shirakawa, K. Yamasaki, T. Hiyama, Synthesis 1998,1544; (f) A. Sophia, E. Karlström, K. Itami, J.-E. Bäckvall, J. Am. Chem. Soc. 2000, 122, 6950; (h) J. Montgomery, Acc. Chem. Res. 2000, 33, 467; (i) R. Giovaninni, P. Knochel, J. Am. Chem. Soc. 1998, 120, 11186; (j) B. H. Lipshutz, Adv. Synth. Catal. 2001, 343, 313; (k) B. H. Lipshutz, G. Bulwo, F. Femandez-Lazaro, S.-K. Kim, R. Lowe, P. Mollard, K. L. Stevens, J. Am. Chem. Soc. 1999, 121, 11664.
[5] (a) M. Tamura, J. K. Kochi, J. Am. Chem. Soc. 1971, 93, 1487; (b) M. Tamura, J. K. Kochi, Synthesis 1971, 93, 303; (c) M. Tamura, J. K. Kochi, J. Organomet. Chem. 1971, 31, 289; (d) M. Tamura, J. K. Kochi, Bull. Chem. Soc. Jpn. 1971, 44, 3063; (e) M. Tamura, J. K. Kochi, Synthesis 1971, 303; (f) J. K. Kochi, Acc. Chem. Res. 1974, 7, 351; (g) S. Neumann, J. K. Kochi, J. Org. Chem. 1975, 40, 599; (h) R. S. Smith, J. K. Kochi, J. Org. Chem. 1976, 41, 502; (i) Des Weiteren: G. Molander, B. Rahn, D. C. Shubert, S. E. Bonde, Tetrahedron Lett. 1983, 24, 5449.
[6] (a) G. Cahiez, S. Marquais, Pure Appl. Chem. 1996, 68, 669; (b) G. Cahiez, S. Marquais, Tetrahedron Lett. 1996, 37, 1773; (c) G. Cahiez, H. Avedissian, Synthesis 1998, 1199; (d) K. Shinokubo, K. Oshima, Eur. J. Org. Chem. 2004, 2081; (e) M. A. Fakhfakh, X. Franck, R. Hocquemiller, B. Figadère, J. Organomet. Chem. 2001, 624, 131; (f) M. Hocek, H. Dvoráková, J. Org. Chem. 2003, 68, 5773; (g) B. Hölzer, R. W. Hoffmann, Chem. Comm. 2003, 732; (h) W. Dohle, F. Kopp, G. Cahiez, P. Knochel, Synlett 2001, 1901; (i) M. Hojo, Y. Murakami, H. Aihara, R. Sakuragi, Y. Baba, A. Hosomi, Angew. Chem. 2001, 113, 641; Angew. Chem. Int.Ed. 2001, 40, 621; (j) M. Nakamura, A. Hirai, E. Nakamura, J. Am. Chem. Soc. 2000, 122, 978; (k)E. Alvarez, T. Cuvigny, C. H.du Penhoat, M. Julia, Tetrahedron 1998, 44, 119; (1) V. Finandanese, G. Marchese, V. Martina, L. Ronzini, Tetrahedron Lett. 1984, 25, 4805.
[7] (a) A. Fürstner, A. Leitner, M. Méndez, H. Krause, J. Am. Chem. Soc. 2002, 124, 13856; (b) A. Fürstner, A. Leitner, Angew. Chem. 2002, 114, 632; Angew.Chem. Int. Ed. 2002, 41, 2003, 42, 308; (c) A. Fürstner, A. Leitner, Angew. Chem. 2003, 115, 320; Angew. Chem. Int. Ed. 2003, 42, 308; (d) G. Seidel, D. Laurich, A. Fürstner, J. Org. Chem. 2004, 69, 3950; (e) B. Scheiper, M. Bonnekessel, H. Krause, A. Fürstner, J. Org. Chem. 2004, 69, 3943; (f) A. Fürstner, D. De Souza, L. Parra-Rapado, J. T. Jensen, Angew. Chem. 2003, 115, 5516; Angew. Chem. Int Ed. 2003, 42, 5355; (g) M. Nakamura, K. Matsuo, S. Ito, E. Nakamura, J. Am. Chem.Soc. 2004, 126, 3686; (h) T. Nagano, T. Hayashi, Org. Lett. 2004, 6, 1297; (i) R. Martin, A. Fürstner, Angew. Chem. 2004, 116, 4045; Angew. Chem. Int. Ed. 2004, 43, 3955.
[8] K. Reddy, P. Knochel, Angew. Chem. 1996, 108, 1812; Angew. Chem. Int. Ed. 1996, 35, 1700.
[9] (a) B. H. Lipshutz, S. Sengupta, Org. React. 1992, 41, 135; (b) R. J. K. Taylor, Organocopper Reagents, Oxford University Press, Oxford, 1994**;** (c) N. Krause, Modern Organocopper Chemistry, Wiley-VCH, Weinheim, 2002**.**
[10] (a) A. Krasovskiy, P. Knochel, Angew. Chem. 2004, 116, 3316; Angew. Chem. Int. Ed. 2004, 43, 3333; (b) Für einen Übersichtsartikel siehe: P. Knochel, W. Dohle, N. Gommermann, F. F. Kneisel, F. Kopp, T. Korn, I. Sapountzis, V. A. Vu, Angew. Chem. 2003, 115, 4438; Angew. Chem. Int. Ed. 2003, 42, 4302.
[11] P. Knochel, M. C. P. Yeh, S. C. Berk, J. Talbert, J. Org. Chem. 1988, 53, 2390.
[12] C. Piazza, P. Knochel, Angew. Chem. 2002, 114, 3263; Angew. Chem. Int. Ed. 2002, 41, 3263.

## Patentansprüche

1. Verfahren zur Herstellung einer Verbindung der allg. Formel **4**
R¹-Ar¹-Ar²-R² (**4**)
indem eine Verbindung der allg. Formel **(1)**
R¹-Ar¹-Cu(Z)MgY **(1)**
oder eine Verbindung der allg. Formel **(5)**
R¹-Ar¹-Cu(Z)Li **(5)**
mit einer Verbindung der allg. Formel **(3)**
R²-Ar²-X **(3)**
unter Einwirkung eines Co- oder Fe-Katalysators in einem Lösungsmittel umgesetzt wird, wobei
X eine für eine nukleophilen Substitution geeignete Abgangsgruppe;
Y Cl, Br, I;
Z CN, Cl, Br, I, SCN, NR¹R², SR¹, PR¹R², Alkyl, Alkinyl;
R¹ und R² unabhängig voneinander ein oder mehrere Substituenten aus H; substituiertem oder unsubstituiertem Aryl oder Heteroaryl, das ein oder mehrere Heteroatome enthält; geradkettigem, verzweigtem oder cyclischen, substituierten oder unsubstituierten Alkyl, Alkenyl, Alkinyl;
Ar¹ und Ar² unabhängig voneinander ein Aryl, kondensiertes Aryl, Heteroaryl oder kondensiertes Heteroaryl, das ein oder mehrere Heteroatome enthält; ein Alkenyl oder Alkinyl;
sein können.

2. Verfahren nach Anspruch 1, wobei die Umsetzung bei einer Temperatur zwischen 0°C und 150°C durchgeführt wird.

3. Verfahren nach Anspruch 1, wobei der Katalysator einen Fe(III)-Komplex, ein Fe(III)-Salz, einen Fe(II)-Komplex, ein Fe(II)-Salz, oder eine reduzierte Form eines Fe-Salzes oder -Komplexes umfasst.

4. Verfahren nach Anspruch 3, wobei der Katalysator Fe(acac)₃ umfasst.

5. Verfahren nach Anspruch 1, wobei der Katalysator einen Co(II)- oder Co(III)-Katalysator, oder eine reduzierte Form eines Co-Salzes oder -Komplexes umfasst.

6. Verfahren nach Anspruch 5, wobei der Katalysator aus der Gruppe, die CoCl₂, CoBr₂, Co(OAc)₂, Co(Bzac)₂, CoBr₂dppe, Co(acac)₂ und Co(acac)₃ umfasst, ausgewählt wird.

7. Verfahren nach Anspruch 6, wobei als Katalysator Co(acac)₂ verwendet wird.

8. Verfahren nach einem der Ansprüche 5 oder 6, wobei zusätzlich Ethen und/oder ein oder mehrere Ethenderivat(e) während der katalytischen Umsetzung zugegeben wird(werden).

9. Verfahren nach Anspruch 8, wobei ein oder mehrere elektronenarme Ethenderivate während der katalytischen Umsetzung zugegeben werden.

10. Verfahren nach Anspruch 8, wobei das Ethylenderivat ein Maleinsäureanhydrid, Tetracyanoethylen, Styrol oder ein Styrolderivat ist.

11. Verfahren nach einem der Ansprüche 8-10, wobei das Ethen oder Ethenderivat in einer Menge von bis zu 50 Mol% bezogen auf die molare Menge der Verbindung (3) zugegeben wird.

12. Verfahren nach einem oder mehreren der Ansprüche 5-8, wobei zusätzlich ein oder mehrere Salze während der katalytischen Umsetzung zugegeben werden.

13. Verfahren nach Anspruch 12, wobei als Salz Tetrabutylammoniumchlorid, Tetrabutylammoniumbromid, Kaliumiodid oder Lithiumiodid während der katalytischen Umsetzung zugegeben wird.

14. Verfahren nach Anspruch 3, wobei X, F, Cl, Br, I, OTf, OTs oder N₂⁺ ist.

15. Verfahren nach Anspruch 5, wobei X, F, Cl, Br, I, OTf, OTs oder N₂⁺ ist.

16. Verfahren nach Anspruch 1, wobei als Lösungsmittel ein polares Lösungsmittel oder Lösungsmittelgemisch verwendet wird.

17. Verfahren nach Anspruch 16, wobei als polares Lösungsmittel ein etherisches Lösungsmittel oder Lösungsmittelgemisch verwendet wird.

18. Verfahren nach Anspruch 1, wobei die Verbindung (1) oder (5) in einem molaren Verhältnis von 0,9 - 5 bezogen auf die molare Menge an Verbindung (3) zugegeben wird.

19. Verfahren nach Anspruch 1, wobei Z, CN ist.

20. Verfahren nach Anspruch 1, wobei R¹ und R² unabhängig voneinander ein substituiertes oder unsubstituiertes C₄-C₂₄ Aryl oder C₃-C₂₄ Heteroaryl, das ein oder mehrere Heteroatome wie B, O, N, S, Se, P enthält; ein geradkettiges oder verzweigtes, substituiertes oder unsubstituiertes C₁-C₂₀ Alkyl, C₁-C₂₀ Alkenyl, C₁-C₂₀ Alkinyl; oder ein substituiertes oder unsubstituiertes C₃-C₂₀ Cycloalkyl sein können.

## Claims

1. Process for preparing a compound of the general formula **4**
R¹-Ar¹-Ar²-R² (**4**)
by reacting a compound of the general formula **(1)**
R¹-Ar¹-Cu(Z)MgY **(1)**
or a compound of the general formula **(5)**
R¹-Ar¹-Cu(Z)Li **(5)**
with a compound of the general formula **(3)**
R²-Ar²-X **(3)**
under the action of a cobalt or iron catalyst in a solvent, where
X is a leaving group suitable for a nucleophilic substitution;
Y is Cl, Br, I;
Z is CN, Cl, Br, I, SCN, NR¹R², SR¹, PR¹R², alkyl, alkynyl;
R¹ and R² are each independently one or more substituents selected from H; substituted or unsubstituted aryl or heteroaryl which contains one or more heteroatoms; straight-chain, branched or cyclic, substituted or unsubstituted alkyl, alkenyl, alkynyl;
Ar¹ and Ar² are each independently an aryl, fused aryl, heteroaryl or fused heteroaryl which contains one or more heteroatoms; an alkenyl or alkynyl.

2. Process according to Claim 1, wherein the reaction is performed at a temperature between 0°C and 150°C.

3. Process according to Claim 1, wherein the catalyst comprises an Fe(III) complex, an Fe(III) salt, an Fe(II) complex, an Fe(II) salt or a reduced form of an iron salt or complex.

4. Process according to Claim 3, wherein the catalyst comprises Fe(acac)₃.

5. Process according to Claim 1, wherein the catalyst comprises a Co(II) or Co(III) catalyst, or a reduced form of a cobalt salt or complex.

6. Process according to Claim 5, wherein the catalyst is selected from the group comprising CoCl₂, CoBr₂, Co(OAc)₂, Co(Bzac)₂, CoBr₂dppe, Co(acac)₂ and Co(acac)₃.

7. Process according to Claim 6, wherein the catalyst used is Co(acac)₂.

8. Process according to either of Claims 5 and 6, wherein ethene and/or one or more ethene derivative(s) is/are additionally added during the catalytic reaction.

9. Process according to Claim 8, wherein one or more electron-deficient ethene derivatives are added during the catalytic reaction.

10. Process according to Claim 8, wherein the ethylene derivative is a maleic anhydride, tetracyanoethylene, styrene or a styrene derivative.

11. Process according to any of Claims 8-10, wherein the ethene or ethene derivative is added in an amount of up to 50 mol% based on the molar amount of the compound (3).

12. Process according to one or more of Claims 5-8, wherein one or more salts are additionally added during the catalytic reaction.

13. Process according to Claim 12, wherein the salt added during the catalytic reaction is tetrabutylammonium chloride, tetrabutylammonium bromide, potassium iodide or lithium iodide.

14. Process according to Claim 3, wherein X is F, Cl, Br, I, OTf, OTs or N₂⁺.

15. Process according to Claim 5, wherein X is F, Cl, Br, I, OTf, OTs or N₂⁺.

16. Process according to Claim 1, wherein the solvent used is a polar solvent or solvent mixture.

17. Process according to Claim 16, wherein the polar solvent used is an ethereal solvent or solvent mixture.

18. Process according to Claim 1, wherein the compound (1) or (5) is added in a molar ratio of 0.9 - 5, based on the molar amount of compound (3).

19. Process according to Claim 1, wherein Z is CN.

20. Process according to Claim 1, wherein R¹ and R² may each independently be a substituted or unsubstituted C₄-C₂₄ aryl or C₃-C₂₄ heteroaryl which contains one or more heteroatoms such as B, O, N, S, Se, P; a straight-chain or branched, substituted or unsubstituted C₁-C₂₀ alkyl, C₁-C₂₀ alkenyl, C₁-C₂₀ alkynyl; or a substituted or unsubstituted C₃-C₂₀ cycloalkyl.

## Revendications

1. Procédé pour la préparation d'un composé de formule générale 4
R¹-Ar¹-Ar²-R² (4)
en ce qu'on transforme un composé de formule générale (1)
R¹-Ar¹-Cu(Z)MgY (1)
ou un composé de formule générale (5)
R¹-Ar¹-Cu(Z)Li (5)
avec un composé de formule générale (3)
R²-Ar²-X (3)
sous l'influence d'un catalyseur au Co ou au Fe dans un solvant, où
X peut représenter un groupe partant approprié pour une substitution nucléophile ;
Y peut signifier Cl, Br, I ;
Z peut signifier CN, Cl, Br, I, SCN, NR¹R², SR¹, PR¹R², alkyle, alcynyle ;
R¹ et R² peuvent signifier, indépendamment l'un de l'autre, un ou plusieurs substituants parmi H ; aryle ou hétéroaryle substitué ou non substitué, qui contient un ou plusieurs hétéroatomes ; alkyle, alcényle, alcynyle linéaire, ramifié ou cyclique, non substitué ou substitué ;
Ar¹ et Ar² peuvent signifier indépendamment l'un de l'autre, un radical aryle, aryle condensé, hétéroaryle ou hétéroaryle condensé, qui contient un ou plusieurs hétéroatomes ; un radical alcényle ou alcynyle.

2. Procédé selon la revendication 1, dans lequel la transformation est réalisée à une température entre 0°C et 150°C.

3. Procédé selon la revendication 1, dans lequel le catalyseur comprend un complexe de Fe (III), un sel de Fe (III), un complexe de Fe (II), un sel de Fe (II) ou une forme réduite d'un sel ou d'un complexe de Fe.

4. Procédé selon la revendication 3, dans lequel le catalyseur comprend Fe(acac)₃.

5. Procédé selon la revendication 1, dans lequel le catalyseur comprend un catalyseur de Co (II) ou Co (III), ou une forme réduite d'un sel ou d'un complexe de Co.

6. Procédé selon la revendication 5, dans lequel le catalyseur est choisi dans le groupe comprenant CoCl₂, CoBr₂, Co(OAc)₂, Co(Bzac)₂, CoBr₂dppe, Co(acac)₂ et Co(acac)₃.

7. Procédé selon la revendication 6, dans lequel on utilise du Co(acac)₂ comme catalyseur.

8. Procédé selon l'une quelconque des revendications 5 ou 6, dans lequel on ajoute en outre de l'éthylène et/ou un ou plusieurs dérivés d'éthylène pendant la transformation catalytique.

9. Procédé selon la revendication 8, dans lequel on ajoute un ou plusieurs dérivés d'éthylène pauvre(s) en électrons pendant la transformation catalytique.

10. Procédé selon la revendication 8, dans lequel le dérivé d'éthylène est un anhydride de l'acide maléique, du tétracyanoéthylène, du styrène ou un dérivé de styrène.

11. Procédé selon l'une quelconque des revendications 8-10, dans lequel l'éthylène ou le dérivé d'éthylène est ajouté en une quantité allant jusqu'à 50% en mole par rapport à la quantité molaire du composé (3).

12. Procédé selon l'une ou plusieurs des revendications 5-8, dans lequel on ajoute en outre un ou plusieurs sels pendant la transformation catalytique.

13. Procédé selon la revendication 12, dans lequel on ajoute comme sel du chlorure de tétrabutylammonium, du bromure de tétrabutylammonium, de l'iodure de potassium, ou de l'iodure de lithium pendant la transformation catalytique.

14. Procédé selon la revendication 3, dans lequel X représente F, Cl, Br, I, OTf, OTs ou N₂⁺.

15. Procédé selon la revendication 5, dans lequel X représente F, Cl, Br, I, OTf, OTs ou N₂⁺.

16. Procédé selon la revendication 1, dans lequel on utilise comme solvant un solvant polaire ou un mélange de solvants.

17. Procédé selon la revendication 16, dans lequel on utilise comme solvant un solvant de type éther ou un mélange de solvants de type éther.

18. Procédé selon la revendication 1, dans lequel le composant (1) ou (5) est ajouté en un rapport molaire de 0,9-5 par rapport à la quantité molaire de composé (3).

19. Procédé selon la revendication 1, dans lequel Z représente CN.

20. Procédé selon la revendication 1, dans lequel R¹ et R² peuvent représenter, indépendamment l'un de l'autre, un radical C₄-C₂₄-aryle non substitué ou substitué ou C₃-C₂₄-hétéroaryle non substitué ou substitué, qui contient un ou plusieurs hétéroatomes tels que B, O, N, S, Se, P ; un radical C₁-C₂₀-alkyle, C₁-C₂₀-alcényle, C₁-C₂₀-alcynyle linéaire ou ramifié, substitué ou non substitué ; ou un radical C₃-C₂₀-cycloalkyle substitué ou non substitué.
